# EUROPÄISCHE PATENTANMELDUNG

(11) **EP 2 907 468 A1**
(43) Veröffentlichungstag der Anmeldung: **19.08.2015**
(21) Anmeldenummer: 15154687.6
(22) Anmeldetag: 11.02.2015
(51) Int. Cl.: A61B 19/00, G05G 1/52

(54) **Bedienelement für eine Positioniervorrichtung einer medizinischen Vorrichtung**

(30) Priorität: 12.02.2014 DE 102014001784
(71) Anmelder: Carl Zeiss Meditec AG, 07745 Jena (DE)
(72) Erfinder: Ernsperger, Stefan, 73479 Ellwangen (DE); Rudolph, Frank, 73431 Aalen (DE)
(74) Vertreter: Carl Zeiss AG - Patentabteilung

(57) **Zusammenfassung**

Die Erfindung betrifft ein Bedienelement 10 für eine Positioniervorrichtung 3 einer medizinischen Vorrichtung 1 zur Bedienung mit dem Mund einer Bedienperson, mit einem Grundkörper 14 und mit einem ersten Sensor 20 zur Entgegennahme eines ersten Bediensignals, welches durch die Bedienperson mit dem Mund ausübbar ist.

Erfindungsgemäß umfasst das Bedienelement eine Steuereinheit 13, die dazu eingerichtet ist, ein erstes Steuersignal für einen ersten Antrieb 6 der Positioniervorrichtung 3 in Abhängigkeit von dem ersten Bediensignal zu erzeugen.

Die Erfindung betrifft ferner eine medizinische Vorrichtung mit einem solchen Bedienelement.

## Beschreibung

Die Erfindung betrifft ein Bedienelement für eine Positioniervorrichtung einer medizinischen Vorrichtung zur Bedienung mit dem Mund einer Bedienperson, mit einem Grundkörper und mit einem ersten Sensor zur Entgegennahme eines ersten Bediensignals, welches durch die Bedienperson mit dem Mund ausübbar ist. Die Erfindung betrifft ferner eine medizinische Vorrichtung mit einem medizinischen Gerät und einer Positioniervorrichtung, an der das medizinische Gerät gehaltert ist, wobei die Positioniervorrichtung mindestens einen Bewegungsfreiheitsgrad und mindestens einen Antrieb zur Einstellung oder Unterstützung einer Position des medizinischen Geräts entlang des mindestens einen Bewegungsfreiheitsgrad aufweist.

Während einer Operation kommen im Regelfall verschiedene medizinische Vorrichtungen zum Einsatz. Durch Bedienvorgänge, die im Verlauf der Operation an den medizinischen Vorrichtungen vorzunehmen sind, wird der Ablauf der Operation häufig unterbrochen oder verzögert. Daraus resultiert eine verlängerte Gesamtdauer der Operation. Dies gilt insbesondere dann, wenn die medizinische Vorrichtung manuell von dem operierenden Arzt selbst bedient wird. Zur Betätigung der medizinischen Vorrichtung muss der Arzt seine Hand aus dem Operationsfeld entfernen und gegebenenfalls in der Hand gehaltene Geräte beiseite legen. Anschließend muss er bei der Wiederaufnahme der Operation Hand und Geräte wieder neu ausrichten, was die Operation verlängert.

Aus dem Stand der Technik ist es bekannt, medizinische Vorrichtungen mit Fußschaltpulten als Bedienelemente zu versehen, damit der operierende Arzt während der Behandlung seine Hände frei halten kann. Ein solches Fußschaltpult ist beispielsweise in der DE 102 2005 013 063 A1 offenbart. Nachteilig an solchen Fußschaltpulten ist jedoch, dass insbesondere bei einer Bedienung im Stehen die Standsicherheit des operierenden Arztes gefährdet ist, da die Fußschaltpulte eine Schwerpunktverlagerung auf ein Standbein des Arztes erfordern. Dadurch ist das Führen medizinischer Instrumente für den Arzt erschwert.

Aus der DE 102 32 688 A1 ist eine weitere medizinische Vorrichtung bekannt, die ein Operationsmikroskop mit einem Mundschalter und ein Stativ umfasst. Durch Beißen auf den Mundschalter wird ein Kontakt geschlossen, so dass ein Strom fließt, durch den eine Arretierung in einem Drehgelenk des Stativs gelöst wird. Anschließend kann das Operationsmikroskop mittels Kraftausübung durch den Arzt auf die Mundstücke im Raum verlagert werden. Nachteilig an dieser Anordnung ist, dass der operierende Arzt die Kräfte zur Bewegung des Operationsmikroskops mit dem Mund direkt übertragen muss, was zu einer Überlastung insbesondere seiner Gesichts-, Hals- und Nackenmuskulatur führen kann.

Eine Aufgabe der vorliegenden Erfindung besteht darin, ein Bedienelement für eine Positioniervorrichtung eines medizinischen Geräts bereitzustellen, das eine möglichst ergonomische und schonende Positionierung ohne Zuhilfenahme der Hände ermöglicht.

Die Aufgabe wird gelöst durch ein Bedienelement mit den Merkmalen des Anspruchs 1.

Erfindungsgemäß umfasst das Bedienelement eine Steuereinheit, die dazu eingerichtet ist, ein erstes Steuersignal für einen ersten Antrieb der Positioniervorrichtung in Abhängigkeit von dem ersten Bediensignal zu erzeugen.

Der erste Sensor des Bedienelements ist dazu ausgebildet, ein erstes Bediensignal entgegenzunehmen, welches von einer Bedienperson mit dem Mund ausgeübt wird. In Abhängigkeit von dem ersten Bediensignal wird in der Steuereinheit ein erstes Steuersignal erzeugt, welches zur Ansteuerung eines ersten Antriebs der Positioniervorrichtung geeignet ist. Auf diese Weise kann durch die Betätigung des ersten Sensors mit dem Mund eine Positionsänderung des medizinischen Geräts in einem Bewegungsfreiheitsgrad der Positioniervorrichtung aktiv herbeigeführt oder unterstützt werden. Die Steuereinheit kann dabei in dem Bedienelement integriert oder separat ausgeführt sein oder als Teil einer überlagerten Steuereinheit der medizinischen Vorrichtung ausgestaltet sein. Begriffe, die im Kontext dieser Erfindung den Ausdruck "steuern" enthalten (wie "Steuereinheit", "Steuersignal" oder "Ansteuerung"), sind nicht auf klassische Steuerungen zu beschränken, sondern können auch Regelungen umfassen.

In einer Ausgestaltung der Erfindung weist das Bedienelement einen zweiten Sensor auf, der zur Entgegennahme eines zweiten Bediensignals eingerichtet ist, welches durch die Bedienperson mit dem Mund ausübbar ist und welches sich von dem ersten Bediensignal unterscheidet, und die Steuereinheit ist dazu eingerichtet, ein zweites Steuersignal für einen zweiten Antrieb der Positioniervorrichtung in Abhängigkeit von dem zweiten Bediensignal zu erzeugen. Der erste Sensor und der zweite Sensor können dabei in einem gemeinsamen Sensorelement oder als zwei separate Sensoren realisiert sein. Wesentlich hierbei ist, dass mittels des gemeinsamen Sensorelements oder mittels des ersten Sensors und des zweiten Sensors zwei unterschiedliche Bediensignale entgegengenommen werden können, die sich bevorzugt durch räumliche Richtungen, in denen sie ausgeübt werden, voneinander unterscheiden. Durch eine Betätigung des zweiten Sensors mit dem Mund kann eine Positionsänderung des medizinischen Geräts durch Ansteuerung eines weiteren Antriebs der Positioniervorrichtung in einem weiteren Bewegungsfreiheitsgrad aktiv herbeigeführt oder unterstützt werden.

In weiteren Ausgestaltungen der Erfindung sind der erste Sensor und/oder der zweite Sensor als Kraft- oder Momentensensoren zur Messung einer Kraft oder eines Moments, insbesondere eines Biegemoments, ausgestaltet. Damit stehen besonders einfache und robuste Sensoren zur Realisierung des Erfindungsgedankens zur Verfügung.

In einer weiteren Ausgestaltung der Erfindung ist eine Bissleiste vorhanden, die in einer ersten Richtung beweglich gegenüber dem Grundkörper gelagert ist, und der erste Sensor ist als Positionssensor zur Messung einer Auslenkung der Bissleiste in der ersten Richtung gegenüber dem Grundkörper ausgebildet. Dadurch ist eine besonders intuitive Bedienung der medizinischen Vorrichtung möglich, da die Vorrichtung derart ausgestaltet werden kann, dass das erste Steuersignal für den ersten Antrieb zu einer Verschiebung des medizinischen Geräts an der Positioniervorrichtung parallel zur Auslenkung der Bissleiste in der ersten Richtung führt.

In einer weiteren Ausgestaltung der Erfindung ist die Bissleiste in einer zweiten Richtung beweglich gegenüber dem Grundkörper gelagert und der zweite Sensor ist als Positionssensor zur Messung einer Auslenkung der Bissleiste in der zweiten Richtung gegenüber dem Grundkörper ausgebildet. Die Bissleiste kann somit von der Bedienperson im Betrieb in einer zweiten, insbesondere von der ersten Richtung verschiedenen Richtung relativ zum Grundkörper der Bedieneinheit verschoben werden. Hierdurch ist die intuitive Bedienbarkeit der medizinischen Vorrichtung weiter verbessert.

In einer weiteren Ausgestaltung der Erfindung ist ein dritter Sensor vorhanden, der zur Entgegennahme eines dritten Bediensignals eingerichtet ist, welches durch die Bedienperson mit dem Mund ausübbar ist, und die Steuereinheit ist dazu eingerichtet, ein Signal zur Ansteuerung einer oder mehrerer Bremsen der Positioniervorrichtung in Abhängigkeit von dem dritten Bediensignal zu erzeugen. Insbesondere lässt sich mit Hilfe des dritten Sensors eine Arretierung eines oder mehrerer Bewegungsfreiheitsgrade der Positioniervorrichtung einrichten oder lösen.

In einer weiteren Ausgestaltung der Erfindung umfasst das Bedienelement eine Befestigungsvorrichtung zur Befestigung des Grundkörpers an der medizinischen Vorrichtung. Dadurch ist es der Bedienperson möglich, nach Befestigung des Bedienelements an der medizinischen Vorrichtung eine durch einen Antrieb der Positioniervorrichtung erzeugte oder unterstützte Bewegung eines an der Positioniervorrichtung gehalterten medizinischen Geräts entlang eines Bewegungsfreiheitsgrads durch Ausübung von Muskelkraft auf das Bedienelement zu unterstützen. Auf diese Weise kann eine Bewegung der medizinischen Vorrichtung an der Positioniervorrichtung bei einem Übergang von einer Arbeitsposition in eine andere Arbeitsposition beschleunigt werden und/oder die Antriebe der Positioniervorrichtung können leistungsschwächer ausgeführt werden.

Eine weitere Aufgabe der vorliegenden Erfindung besteht darin, eine medizinische Vorrichtung mit einem Bedienelement bereitzustellen.

Die Aufgabe wird durch eine medizinische Vorrichtung mit den Merkmalen des Anspruchs 9 gelöst. Erfindungsgemäß umfasst die medizinische Vorrichtung ein Bedienelement nach einem der Ansprüche 1 bis 8.

Im Nachfolgenden wird die Erfindung anhand der Zeichnungen näher erläutert. Hierbei zeigen im Einzelnen
- Fig. 1: ein Ausführungsbeispiel einer erfindungsgemäßen medizinischen Vorrichtung und
- Fig. 2: verschiedene Ausführungsbeispiele von erfindungsgemäßen Bedienelementen

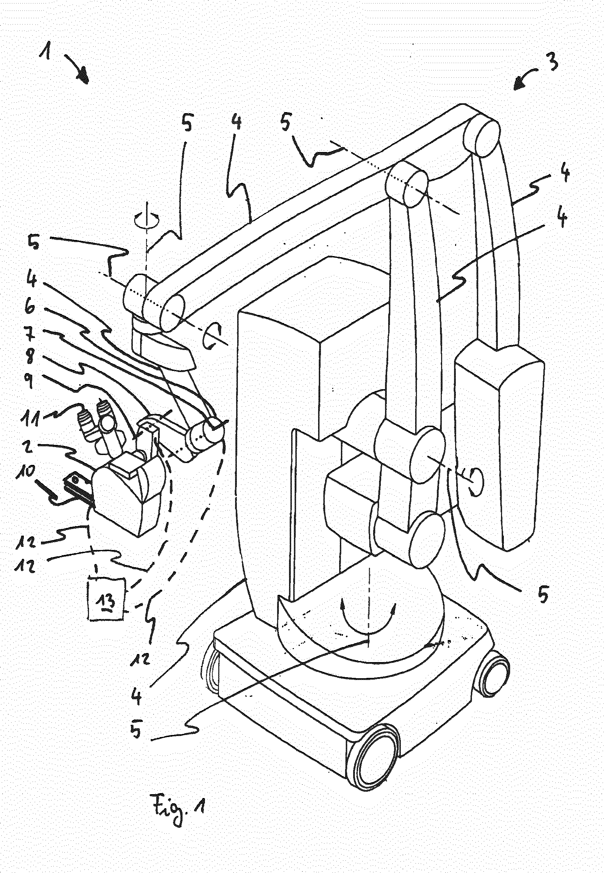

In Figur 1 ist eine erfindungsgemäße medizinische Vorrichtung 1 dargestellt, die ein medizinisches Gerät in Form eines Operationsmikroskops 2 umfasst, das an einer Positioniervorrichtung in Form eines Stativs 3 gehaltert ist.

In diesem Ausführungsbeispiel ist das Stativ 3 aus mehreren seriell und parallel angeordneten Tragarmen 4 aufgebaut, die über Gelenkverbindungen 5 miteinander verbunden sind. Einem ersten Drehgelenk 7 ist ein erster Antrieb 6 zugeordnet und einem zweiten Drehgelenk 9 ein zweiter Antrieb 8. Mit dem ersten Antrieb 6 lässt sich ein Drehmoment um eine Drehachse des ersten Drehgelenks 7 erzeugen, so dass eine Winkelauslenkung zwischen den Tragarmen, die durch das erste Drehgelenk 7 miteinander verbunden sind, einstellbar ist. Entsprechend ist mit dem zweiten Antrieb 8 ein Drehmoment um eine Drehachse des zweiten Drehgelenks 9 ausübbar, so dass eine Winkelauslenkung zwischen den Tragarmen, die durch das zweite Drehgelenk 9 miteinander verbunden sind, einstellbar ist.

An dem Operationsmikroskop 2 ist ein Bedienelement 10 angebracht, welches von einer Bedienperson mit dem Mund gefasst werden kann. Unter dem Begriff "Mund" sind dabei alle Körperteile im und um einen Mundraum der Bedienperson zu verstehen, beispielsweise Zähne, Zunge, Gaumen, Kiefer oder Lippen. In diesem Ausführungsbeispiel ist das Bedienelement 10 dazu ausgebildet, von der Bedienperson mit den Zähnen gehalten und bedient zu werden.

Das Bedienelement 10 ist derart an dem Operationsmikroskop angeordnet, dass die Bedienperson ein Operationsfeld mit den Augen durch Okulare 11 des Operationsmikroskops 2 betrachten kann, während sie das Bedienelement mit den Zähnen oder einem anderen Teil des Munds hält.

Mit Hilfe des Bedienelements 10 ist die Bedienperson in der Lage, eine Position des Operationsmikroskops 2 in mindestens einem Freiheitsgrad einzustellen. Das Funktionsprinzip wird nachfolgend exemplarisch anhand einer Drehung um eine Drehachse des ersten Drehgelenks 7 beschrieben. Hierfür übt die Bedienperson wie nachfolgend noch genauer beschrieben mit dem Mund auf einen ersten Sensor des Bedienelements 10 ein erstes Bediensignal aus. Das erste Bediensignal wird über eine Signalübertragungsleitung 12, beispielsweise ein Kabel, eine Platinenleitung oder eine Drahtlosverbindung, an eine Steuereinheit 13 übermittelt. In der Steuereinheit 13 wird unter Berücksichtigung des ersten Bediensignals ein erstes Steuersignal bestimmt, welches über eine weitere Signalübertragungsleitung 12 an einen ersten Antrieb 6 des Stativs übermittelt wird, der dem ersten Drehgelenk 7 zugeordnet ist. Über den ersten Antrieb 6 wird ein Drehmoment um die Drehachse eingestellt, so dass das Operationsmikroskop 2 um die Drehachse in die gewünschte Position verschwenkt wird.

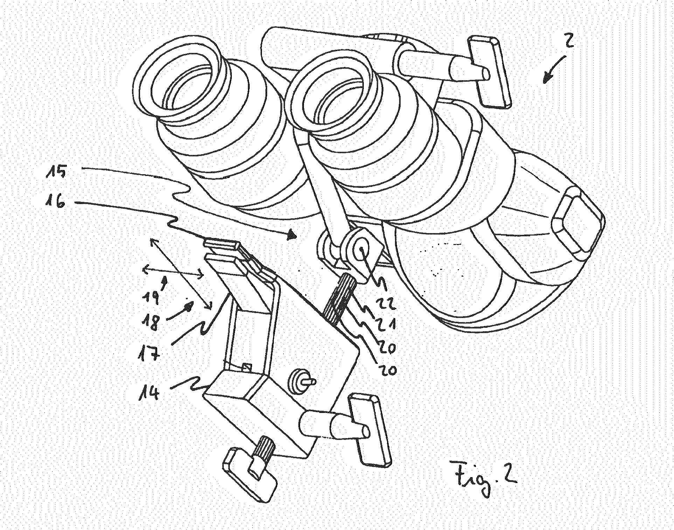

In Figur 2 ist ein Ausführungsbeispiel für ein erfindungsgemäßes Bedienelement 10 in größerem Detail dargestellt.

Das Bedienelement 10 umfasst einen Grundkörper 14, der über eine Befestigungsvorrichtung 15 an dem Operationsmikroskop 2 befestigt ist. An dem Grundkörper 14 ist eine erste Bissleiste 16 ausgebildet, die mit dem Grundkörper fest verbunden ist und auf die die Bedienperson mit den Zähnen eines Kiefers (in diesem Fall des Oberkiefers) beißen kann.

Unterhalb der ersten Bissleiste 16 ist eine zweite Bissleiste 17 angeordnet, die in diesem Ausführungsbeispiel in zwei Richtungen entlang der Doppelpfeile 18, 19 beweglich gegenüber dem Grundkörper 14 gelagert ist. Die zweite Bissleiste 17 steht während des Betriebs in Kontakt mit den Zähnen des anderen Kiefers der Bedienperson (in diesem Fall des Unterkiefers). Durch Verschieben des Unterkiefers relativ zum Oberkiefer kann die Bedienperson die zweite Bissleiste 17 in Richtung der beiden Doppelpfeile 18, 19 auslenken.

Mit Hilfe eines in Fig. 2 nicht näher dargestellten ersten Sensors wird ein erstes Bediensignal in Form einer Auslenkung der zweiten Bissleiste 17 in Richtung des ersten Doppelpfeils 18 ermittelt. Ein ebenfalls nicht näher dargestellter zweiter Sensor dient zur Ermittlung eines zweiten Bediensignals in Form einer Auslenkung der zweiten Bissleiste 17 in Richtung des zweiten Doppelpfeils 19. Der ersten Sensor und der zweite Sensor sind bevorzugt als Positionssensoren ausgestaltet, worunter im Kontext dieser Erfindung sowohl einfache Schalter, die ab einer definierten Auslenkung der zweiten Bissleiste 17 geschaltet werden, als auch Sensoren zur Messung einer Auslenkungswegstrecke, einer Auslenkungsgeschwindigkeit, einer Auslenkungsbeschleunigung oder einer anderen, zur direkten oder indirekten Bestimmung einer Auslenkung geeigneten physikalischen Größe, zu verstehen sind.

Die Erfindung wurde vorstehend anhand einer zweifach translatorisch gegenüber dem Grundkörper gelagerten zweiten Bissleiste erläutert. Sie ist jedoch nicht auf eine solche Ausgestaltung beschränkt. Ohne den Rahmen der Erfindung zu verlassen kann die Lagerung der Bissleiste gegenüber dem Grundkörper sowohl translatorisch (als Schubgelenk) als auch rotatorisch (als Drehgelenk) oder als Kombination aus einer translatorischer und einer rotatorischer Lagerung (als Dreh-Schubgelenk) ausgeführt sein. Es ist ohne weiteres auch vorstellbar, die zweite Bissleiste nur einfach gelagert oder mehr als zweifach gegenüber dem Grundkörper gelagert auszugestalten. Anzahl und Art der Sensoren sind an die Form der Lagerung in geeigneter Weise anzupassen.

Anhand von Figur 2 wird nachfolgend ein weiteres Ausführungsbeispiel eines erfindungsgemäßen Bedienelements 10 erläutert. Hierbei ist auch die zweite Bissleiste 17 fest mit dem Grundkörper 14 verbunden. Alternativ kann die zweite Bissleiste in diesem Ausführungsbeispiel auch entfallen, so dass die Bedienperson lediglich die erste Bissleiste 16 mit dem Mund greift. Anstelle von Positionssensoren zur Messung einer Auslenkung der zweiten Bissleiste sind in diesem Ausführungsbeispiel zwei Sensoren in Form von Dehnmessstreifen 20 zur Messung von Biegespannungen an einer Befestigungsstange 21 der Befestigungsvorrichtung 15 angeordnet. Im Betrieb fasst die Bedienperson den Grundkörper über die Bissleiste mit dem Mund und übt eine Kraft und/oder ein Drehmoment auf den Grundkörper auf. Die Kraft und/oder das Drehmoment führt zu einer geringen Verformung beziehungsweise Biegung der Befestigungsstange, die über die Dehnmessstreifen 20 als Bediensignal detektiert wird. Das Bediensignal wird wie im ersten Ausführungsbeispiel an die Steuereinheit weitergeleitet, in der ein Steuersignal für die Antriebe unter Berücksichtigung des Bediensignals erzeugt wird. Das Bedienelement kann mit einem oder mit mehreren Dehnmessstreifen zur Messung eines oder mehrerer Bediensignale ausgestattet sein.

Ohne Einschränkung der Allgemeinheit können anstelle der oben beschriebenen Dehnmessstreifen auch andere Kraft- oder Momentensensoren zur direkten oder indirekten Messung einer von der Bedienperson auf den Grundkörper ausgeübten Kraft oder eines Drucks oder eines Moments in einem erfindungsgemäßen Bedienelement verwendet werden.

Ebenfalls anhand von Figur 2 wird ein drittes Ausführungsbeispiel eines erfindungsgemäßen Bedienelements beschrieben. Das dritte Ausführungsbeispiel unterscheidet sich von dem ersten Ausführungsbeispiel dadurch, dass die Positionssensoren als Dreh- oder Kippsensoren in einem Gelenk 22 der Befestigungsvorrichtung 15 ausgestaltet sind. Die Positionssensoren befinden sich im Betrieb des Bedienelements also nicht im Mundraum der Bedienperson wie im ersten Ausführungsbeispiel, sondern außerhalb desselbigen.

Die Erfindung wurde vorstehend anhand von zwei Antrieben an zwei Drehgelenken des Stativs beschrieben. Sie ist jedoch nicht auf eine solche Anordnung zu beschränken. Ohne den Rahmen der vorliegenden Erfindung zu verlassen können beliebige Arten von Gelenkverbindungen (beispielsweise Schubgelenke, Drehschubgelenke oder Schraubgelenke) zwischen Tragarmen des Stativs und beliebige Kombinationen von Antrieben an den Gelenkverbindungen des Stativs vorgesehen sein. Entscheidend ist, dass mindestens einer Gelenkverbindung des Stativs ein Antrieb zugeordnet ist, mit dessen Hilfe ein Drehmoment um eine Drehachse oder eine Kraft in Richtung einer Gelenkachse ausgeübt werden kann, so dass eine Auslenkung (beispielsweise ein Winkel oder eine Wegstrecke) zwischen den Tragarmen, die durch die Gelenkverbindung miteinander verbunden sind, einstellbar ist.

Bevorzugt ist das Stativ 3 derart aufgebaut, dass das Operationsmikroskop 2 in sechs Freiheitsgraden (drei rotatorische Freiheitsgrade und drei translatorische Freiheitsgrade) innerhalb eines Arbeitsraums ausgerichtet werden kann. Ohne Einschränkung der Allgemeinheit sind aber auch Ausführungen des Stativs mit weniger oder mehr als sechs Freiheitsgraden denkbar.

In allen Ausführungsbeispielen kann ein dritter Sensor in Form eines Schalters vorgesehen sein, der zur Entgegennahme eines dritten Bediensignals, welches mit den Zähnen, den Lippen oder anderen Teilen des Munds der Bedienperson erzeugbar ist, ausgestaltet ist. Das dritte Bediensignal wird ebenfalls an die Steuereinheit 13 übermittelt und dort in ein Signal oder in mehrere Signale zur Arretierung oder zum Lösen einer Bremse oder mehrerer Bremsen in den Gelenkverbindungen 5, 7, 8 des Stativs 3 umgewandelt. Damit ist eine sichere Fixierung des Operationsmikroskops 2 in einer Arbeitsposition gewährleistet. Bevorzugt lassen sich mit Hilfe des dritten Sensors diejenigen Bremsen des Stativs 3 lösen, die den Antrieben 6, 8 des Stativs zugeordnet sind, die sich über den ersten Sensor und den zweiten Sensor (und gegebenenfalls weitere Sensoren) des Bedienelements 10 ansteuern lassen. Auf diese Weise kann eine Bedienperson bei einem Verfahren des Operationsmikroskops 2 von einer Arbeitsposition in eine andere zunächst durch Betätigung des dritten Sensors die Bremsen lösen und anschließend das Operationsmikroskop 2 ohne große körperliche Anstrengung durch Betätigung des ersten Sensors und/oder des zweiten Sensors und/oder gegebenenfalls weiterer Sensoren in die neue Arbeitsposition verfahren.

In einem weiteren, nicht dargestellten Ausführungsbeispiel ist das Stativ mit den Antrieben derart ausgeführt, dass in erster Linie nur Drehwinkel des Operationsmikroskop mit Hilfe der Sensoren des Bedienelements und der Antriebe einstellbar sind. Translatorische Positionen des Operationsmikroskops im Arbeitsraum lassen sich entweder manuell oder auf herkömmliche Weise motorisch unterstützt einstellen. Diese Ausführungsform trägt dem Umstand Rechnung, dass in vielen Operationssituationen das Operationsmikroskop nur in geringem Umfang translatorisch und in viel größerem Umfang rotatorisch bewegt wird.

In wiederum einem weiteren, nicht dargestellten Ausführungsbeispiel sind die Antriebe derart dimensioniert, dass ihre Leistung alleine nicht ausreicht, um aus allen Arbeitspositionen des Operationsmikroskops im Arbeitsraum heraus eine Bewegung des Operationsmikroskops in Richtung einer neuen Arbeitsposition herbeizuführen. Die Antriebe unterstützen in diesem Fall lediglich die Bedienperson beim Verfahren des Operationsmikroskops von einer Arbeitsposition in eine andere.

Die Erfindung wurde vorstehend anhand von Bedienelementen beschrieben, die fest mit einem Operationsmikroskop verbunden sind. Ohne Einschränkung der Allgemeinheit kann das Bedienelement jedoch auch losgelöst von dem Operationsmikroskop ausgeführt sein, so dass eine Bedienperson es beispielsweise vollständig oder zumindest teilweise im Mundraum aufnehmen kann. Die Signalübertragung von dem Bedienelement zu der Steuereinheit erfolgt in diesem Fall beispielsweise drahtlos.

## Patentansprüche

1. Bedienelement (10) für eine Positioniervorrichtung (3) einer medizinischen Vorrichtung (1) zur Bedienung mit dem Mund einer Bedienperson,
mit einem Grundkörper (14) und
mit einem ersten Sensor (20) zur Entgegennahme eines ersten Bediensignals, welches durch die Bedienperson mit dem Mund ausübbar ist,
**dadurch gekennzeichnet, dass**
das Bedienelement eine Steuereinheit (13) umfasst, die dazu eingerichtet ist, ein erstes Steuersignal für einen ersten Antrieb (6) der Positioniervorrichtung (3) in Abhängigkeit von dem ersten Bediensignal zu erzeugen.

2. Bedienelement nach Anspruch 1,
**dadurch gekennzeichnet, dass**
das Bedienelement (10) einen zweiten Sensor (23) aufweist, der zur Entgegennahme eines zweiten Bediensignals eingerichtet ist, welches durch die Bedienperson mit dem Mund ausübbar ist und welches sich von dem ersten Bediensignal unterscheidet, und dass
die Steuereinheit (13) dazu eingerichtet ist, ein zweites Steuersignal für einen zweiten Antrieb (8) der Positioniervorrichtung (3) in Abhängigkeit von dem zweiten Bediensignal zu erzeugen.

3. Bedienelement nach Anspruch 1,
**dadurch gekennzeichnet, dass**
der erste Sensor (20) als Kraft- oder Momentensensor ausgestaltet ist.

4. Bedienelement nach Anspruch 2,
**dadurch gekennzeichnet, dass**
der zweite Sensor (23) als Kraft- oder Momentensensor ausgestaltet ist.

5. Bedienelement nach einem der Ansprüche 1, 2 oder 4,
**dadurch gekennzeichnet, dass**
eine Bissleiste (17) vorhanden ist, die in einer ersten Richtung (18) beweglich gegenüber dem Grundkörper (14) gelagert ist und dass der erste Sensor als Positionssensor zur Messung einer Auslenkung der Bissleiste (17) in der ersten Richtung (18) gegenüber dem Grundkörper (14) ausgebildet ist.

6. Bedienelement nach Anspruch 5, rückbezogen auf Anspruch 2,
**dadurch gekennzeichnet, dass**
die Bissleiste (17) in einer zweiten Richtung (19) beweglich gegenüber dem Grundkörper (14) gelagert ist und dass der zweite Sensor als Positionssensor zur Messung einer Auslenkung der Bissleiste (17) in der zweiten Richtung (19) gegenüber dem Grundkörper (14) ausgebildet ist.

7. Bedienelement nach einem der Ansprüche 1 bis 6,
**dadurch gekennzeichnet, dass**
ein dritter Sensor vorhanden ist, der zur Entgegennahme eines dritten Bediensignals eingerichtet ist, welches durch die Bedienperson mit dem Mund ausübbar ist, und dass die Steuereinheit (13) dazu eingerichtet ist, ein Signal zur Ansteuerung einer oder mehrerer Bremsen der Positioniervorrichtung (3) in Abhängigkeit von dem dritten Bediensignal zu erzeugen.

8. Bedienelement nach einem der Ansprüche 1 bis 7,
**dadurch gekennzeichnet, dass**
das Bedienelement (10) eine Befestigungsvorrichtung (15) zur Befestigung des Grundkörpers (14) an der medizinischen Vorrichtung (1) umfasst.

9. Medizinische Vorrichtung (1) mit einem medizinischen Gerät (2) und einer Positioniervorrichtung (3), an der das medizinische Gerät (2) gehaltert ist, wobei die Positioniervorrichtung (3) mindestens einen Bewegungsfreiheitsgrad und mindestens einen Antrieb (5, 6, 8) zur Einstellung oder Unterstützung einer Position des medizinischen Geräts (2) entlang des mindestens einen Bewegungsfreiheitsgrad aufweist,
**dadurch gekennzeichnet, dass**
die medizinische Vorrichtung (1) ein Bedienelement (10) nach einem der Ansprüche 1 bis 8 umfasst.

10. Medizinische Vorrichtung nach Anspruch 9,
**dadurch gekennzeichnet, dass**
die Positioniervorrichtung (3) mit dem mindestens einen Antrieb (5, 6, 8) derart ausgeführt ist, dass nur Drehwinkel des Operationsmikroskops mit Hilfe des ersten Sensors (20) und/oder des zweiten Sensors (23) des Bedienelements (10) und des mindestens einen Antriebs einstellbar sind.

11. Medizinische Vorrichtung nach Anspruch 10,
**dadurch gekennzeichnet, dass**
translatorische Positionen des Operationsmikroskops im Arbeitsraum manuell oder motorisch unterstützt einstellbar sind.
